# FASCICULE DE BREVET EUROPEEN

(11) **EP 0 595 974 B1**
(45) Date de publication et mention de la délivrance du brevet: **19.03.1997**
(21) Numéro de dépôt: 92916342.6
(22) Date de dépôt: 15.07.1992
(51) Int. Cl.: G01B 11/24, G01B 1/00, A43D 1/02, A61B 5/103

(54) **PROCEDE DE MESURE TRIDIMENSIONNELLE, SANS CONTACT, DE L'ENVELOPPE D'UN OBJECT, NOTAMMENT UN PIED, ET APPAREIL DE MESURE PERMETTANT LA MISE EN OEUVRE DU PROCEDE**
DREI-DIMENSIONALMESSVERFAHREN, OHNE KONTAKT, DER UMHÜLLUNG EINES OBJEKTES, INSBESONDERE EINES FUSSES, UND MESSAPPARAT ZUR DURCHFÜHRUNG DES VERFAHRENS
CONTACT-FREE METHOD FOR TRIDIMENSIONAL MEASUREMENT OF THE ENVELOPE OF AN OBJECT, PARTICULARLY A FOOT, AND MEASURING APPARATUS FOR IMPLEMENTING SUCH METHOD

(30) Priorité: 15.07.1991 FR 9108895
(43) Date de publication de la demande: 11.05.1994
(73) Titulaire: Huberty, Stéphane, B-1180 Bruxelles (BE); IDEAS, B-1180 Bruxelles (BE)
(72) Inventeur: HUBERTY, Stéphane, F-30110 Branoux (FR)
(74) Mandataire: Casalonga, Axel
(86) Numéro de dépôt international: FR9200679
(87) Numéro de publication internationale: WO9302336

(56) Documents cités:
- EP-A- 0 014 022
- WO-A-90/05345
- DE-A- 3 508 730
- FR-A- 2 580 487
- US-A- 4 745 290
- PATENT ABSTRACTS OF JAPAN vol. 7, no. 156 (P-209)(1301) 8 Juillet 1983 ; & JP-A-58 066 004

## Description

La présente invention concerne un procédé de mesure tridimensionnelle, sans contact, de l'enveloppe d'un objet, et notamment le pied d'une personne, en vue de la réalisation d'une chaussure sur mesure, orthopédique ou non.

Ce procédé de mesure peut bien entendu s'appliquer à tous objets tridimensionnels.

La présente invention concerne également un appareil de mesure utilisé pour la mise en oeuvre du procédé.

Les différentes étapes de fabrication d'une chaussure sur mesure consistent à mesurer le pied, à fabriquer, en fonction des mesures prises, un gabarit appelé couramment et ci-après "forme", à mesurer cette forme et, en fonction des mesures prises, à découper différentes pièces appelées "patrons" dans un matériau, par exemple du cuir, puis enfin à assembler ces différentes pièces, principalement par couture et collage, en prenant appui sur la forme.

Le procédé de l'invention s'applique donc à la première étape de la réalisation d'une chaussure sur mesure, c'est-à-dire à la mesure d'un pied.

Un procédé de mesure connu consiste à mesurer le pied manuellement en utilisant un mètre ruban. Ce procédé présente beaucoup d'inconvénients. En effet, il y a contact entre le mètre ruban et le pied, la tension et la localisation du mètre varient, ce qui introduit des facteurs de variabilité de la mesure, le nombre de mesures effectuées est limité à quelques périmètres et contours (entre 5 et 10), et enfin la situation précise d'une mesure dans l'espace est impossible, les informations sur l'enveloppe tridimensionnelle étant alors quasi nulles car on ne mesure que des périmètres et des projections.

Un autre procédé de mesure utilisé actuellement, mais sans contact, consiste à mesurer le pied par la technique de "moiré" ou du plan laser unique couplé à une prise de vue par caméra, et relié à un ordinateur qui détermine les trois coordonnées de chaque point de mesure. Ce procédé de mesure, par digitalisation, est plus précis que le procédé de mesure manuel, mais le pied est mesuré en décharge, c'est-à-dire qu'il ne repose pas sur le sol. Cette mesure en décharge permet la mesure directe de la plante du pied, mais présente un inconvénient important, car la forme du pied varie lorsqu'il est en charge (position debout de la personne) et les mesures effectuées en décharge ne correspondent plus à l'enveloppe du pied en charge.

Ce procédé fournit donc des mesures fausses, et de plus, il impose un nombre de points de vue plus important pour mesurer le dessus et la plante du pied.

US 4 745 290 décrit un procédé consistant à mesurer successivement les parties supérieures droite et gauche d'un pied d'une personne en vue de fabriquer des chaussures. Cependant, ce document ne permet pas d'arriver à un accord parfait entre le pied et la chaussure réalisée.

WO-A-90/05 345 concerne un procédé de mesure de la plante du pied d'une personne en vue de fabriquer, non pas des chaussures, mais des semelles orthopédiques. Selon ce procédé le pied de la personne repose sur une vitre permettant la mesure optique de la plante du pied.

Le résumé en langue anglaise de JP-A-58 066 004 décrit un dispositif permettant de mesurer la forme d'un objet immergé dans une résine liquide durcissable, ce qui est totalement inadapté à la mesure de l'enveloppe d'un pied en vue de réaliser une chaussure.

La présente invention a pour but d'assurer un accord parfait entre l'enveloppe du pied et la chaussure réalisée, ce qui procure un grand confort à l'utilisateur. Ce but est atteint en effectuant la mesure du pied en charge, donc dans les conditions réelles d'utilisation des chaussures et avec moins de points de vue, ce qui diminue le coût de l'appareil de mesure.

Telle que revendiquée, la présente invention a pour objet un procédé de mesure tridimensionnelle, sans contact, de l'enveloppe, d'une partie du corps d'une personne, et plus particulièrement d'un pied, par digitalisation à l'aide d'un moyen de capture relié à un calculateur.

Selon l'invention, ce procédé consiste à poser ladite partie sur un support déformable rémanent permettant de supporter ladite partie en charge, à mesurer la partie en deux temps, un premier temps correspondant à la mesure de la région supérieure de la partie par ledit moyen de capture occupant une première position repérable par rapport au support et, après avoir retiré la partie du support, un deuxième temps correspondant à la mesure de l'empreinte de la partie sur le même support par ledit moyen de capture occupant une deuxième position également repérable par rapport à ce même support, les deux positions du moyen de capture étant identiques ou différentes, les deux mesures étant alors traitées pour reconstituer l'enveloppe globale de ladite partie.

Selon une caractéristique de l'invention, ce procédé consiste à effectuer deux prises de vue pour le premier temps de mesure et une prise de vue pour le deuxième temps de mesure. De manière préférentielle, les deux prises de vue du premier temps de mesure sont, soit une prise de vue postérieure, supérieure et axiale et une prise de vue antérieure, supérieure et axiale, soit une prise de vue postérieure, supérieure et latérale et une prise de vue postérieure, supérieure et médiale.

A titre optionnel, dans le cas des deux prises de vue, l'une latérale et l'autre médiate, ce procédé consiste à effectuer une troisième prise de vue, cette prise de vue étant supérieure, axiale, antérieure ou postérieure.

Selon une autre caractéristique de l'invention, ce procédé consiste à effectuer une prise de vue pour le deuxième temps de mesure, cette prise de vue étant supérieure, axiale, antérieure ou postérieure.

Le deuxième temps de mesure peut également s'effectuer selon deux prises de vue, deux prises de vue supérieure, axiale, l'une antérieure et l'autre postérieure, ou une prise de vue postérieure, supérieure et latérale et une prise de vue postérieure, supérieure et médiale.

Telle que revendiquée, la présente invention a également pour objet un appareil de mesure tridimensionnelle de l'enveloppe d'une partie du corps humain par digitalisation à l'aide d'un moyen de capture relié à un calculateur.

Selon l'invention, cet appareil comporte un support déformable rémanent recevant ladite partie à mesurer en charge et le moyen de capture occupe au moins une position repérable par rapport au support.

Le moyen de capture peut comporter un projecteur et une caméra qui forment entre eux un angle déterminé, ce projecteur et cette caméra étant placés l'un par rapport à l'autre à une distance déterminée.

Les axes principaux du projecteur et de la caméra convergent en un point proche ou situé dans l'objet à mesurer. Les projecteurs sont préférentiellement des projecteurs de multiples plans lumineux. L'intersection de ces plans lumineux avec l'objet à mesurer dessinent sur celui-ci des intersections des plans lumineux avec l'enveloppe de l'objet. Un ou plusieurs de ces plans est singularisé et permet de situer de proche en proche les plans suivants. La caméra observe ces intersections et transmet celles-ci au calculateur. Etant donné que l'on connait l'angle et la distance qui relient le projecteur et chacun de ces plans avec la caméra, le calculateur peut déterminer par triangulation les trois dimensions de l'objet mesuré dans le champ de vision du capteur.

Selon un premier mode de réalisation, l'appareil comporte un seul capteur composé d'un projecteur et d'une caméra, ce capteur étant déplaçable pour effectuer des prises de vue d'au moins deux points de vue différents.

Selon un deuxième mode de réalisation, cet appareil comporte plusieurs capteurs fixes dans l'espace.

Selon un premier exemple de réalisation du deuxième mode, les deux capteurs sont situés dans l'axe du support et effectuent, l'un une prise de vue postérieure et supérieure, tandis que l'autre effectue une prise de vue antérieure et supérieure.

Selon un deuxième exemple de réalisation du deuxième mode, les deux capteurs sont désaxés par rapport à l'axe du support, l'un effectuant une prise de vue postérieure, supérieure et latérale, tandis que l'autre effectue une prise de vue postérieure, supérieure et médiale.

Il est décrit ci-après à titre d'exemple et en référence aux dessins annexés, un appareil selon l'invention, mesurant un objet qui est dans ce cas, un pied.
La figure 1 montre l'appareil de l'invention muni de deux capteurs situés dans l'axe du support et mesurant le dessus du pied installé sur un support déformable, ce qui correspond au premier temps de mesure;
la figure 2 montre l'appareil de l'invention avec les mêmes capteurs mesurant l'empreinte du pied sur le support déformable, ce qui correspond au deuxième temps de mesure.
Les figures 3 et 4 correspondent respectivement aux figures 1 et 2, mais dans ce cas les capteurs sont placés de chaque côté de l'axe du support, de manière désaxée.

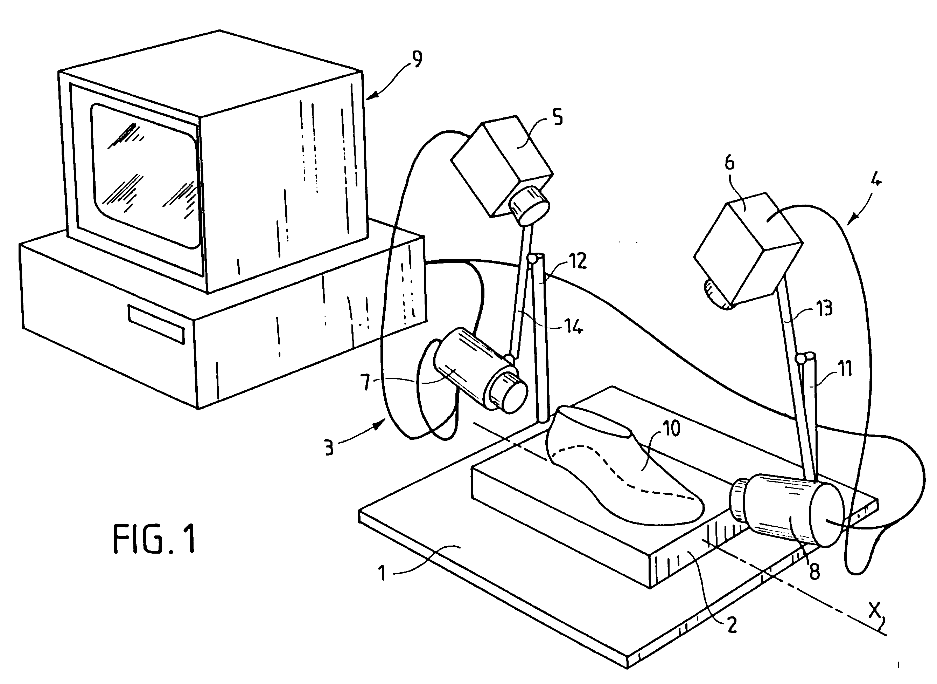

Dans la figure 1, l'appareil comporte une embase I, un support déformable 2, deux capteurs 3, 4 composés chacun d'une caméra, respectivement 5, 6 et d'un projecteur, respectivement 7, 8 et enfin un calculateur 9, les deux caméras et éventuellement les deux projecteurs étant reliés au calculateur.

Le support déformable 2 repose sur l'embase 1, et un pied 10 représenté de manière schématique repose sur le support 2 qui est constitué d'une matière rémanante, comme par exemple des microbilles ou une mousse.

L'embase 1 reçoit à ses deux extrémités deux potences 11, 12, la potence Il porte un bras 13 aux extrémités duquel sont installés la caméra 5 et le projecteur 7 du capteur 3, et la potence 12 porte un bras 14 aux extrémités duquel sont installés la caméra 6 et le projecteur 8 du capteur 4.

Les deux capteurs 3, 4 sont situés dans l'axe principal X du support 1, l'axe du pied étant placé idéalement sur cet axe.

Le talon du pied est dirigé vers le capteur 3, de sorte que la caméra 5 réalise une prise de vue postérieure, supérieure et axiale et la caméra 6 réalise une prise de vue antérieure, supérieure et axiale.

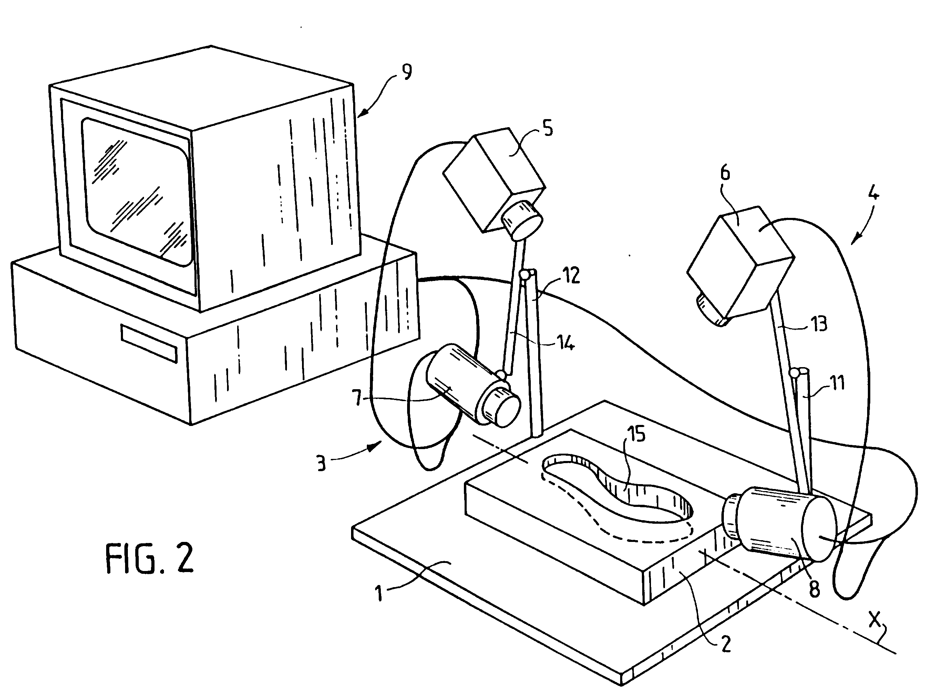

La figure 2 est identique à la figure 1 en ce qui concerne l'emplacement des capteurs. Le pied a été retiré du support déformable 2 pour ne laisser qu'une empreinte 15. Les deux caméras 5, 6 effectuent alors chacune une mesure de l'empreinte.

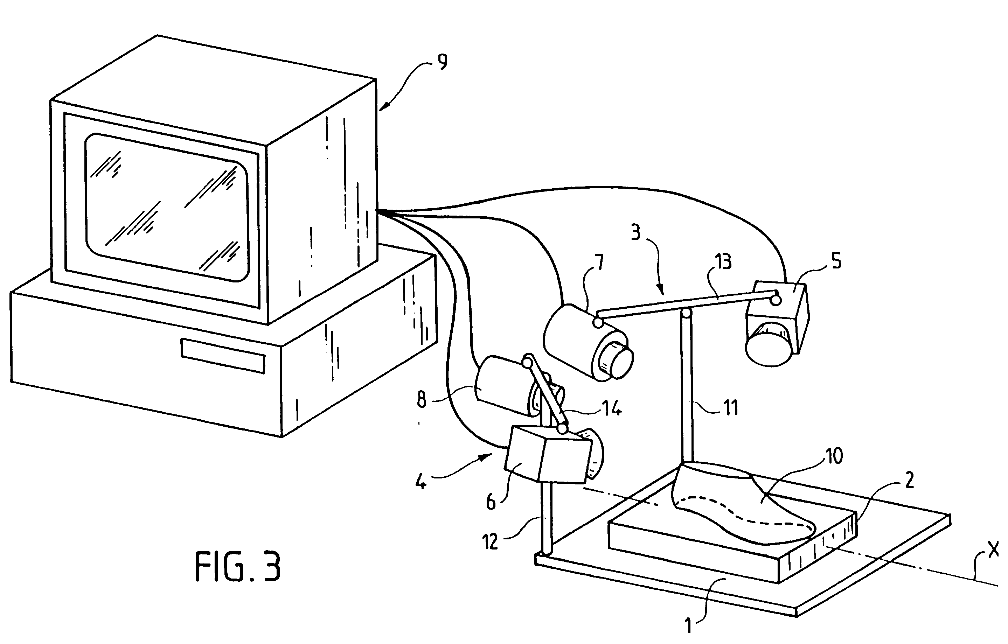

La figure 3 montre, comme la figure 1, la mesure du dessus du pied, mais dans cette figure 3, les capteurs sont latéraux, c'est-à-dire placés de part et d'autre, et par exemple symétrique par rapport à l'axe X. Le bras 13 est dans ce cas horizontal et bien entendu il peut prendre toutes les orientations possibles. Le talon du pied est dirigé vers les deux capteurs 3, 4, de sorte que la caméra 5 réalise une prise de vue postérieure, supérieure et médiale (intérieur d'un pied droit dans le cas de la figure), et la caméra 6 réalise une prise de vue postérieure, supérieure et latérale (extérieur d'un pied droit).

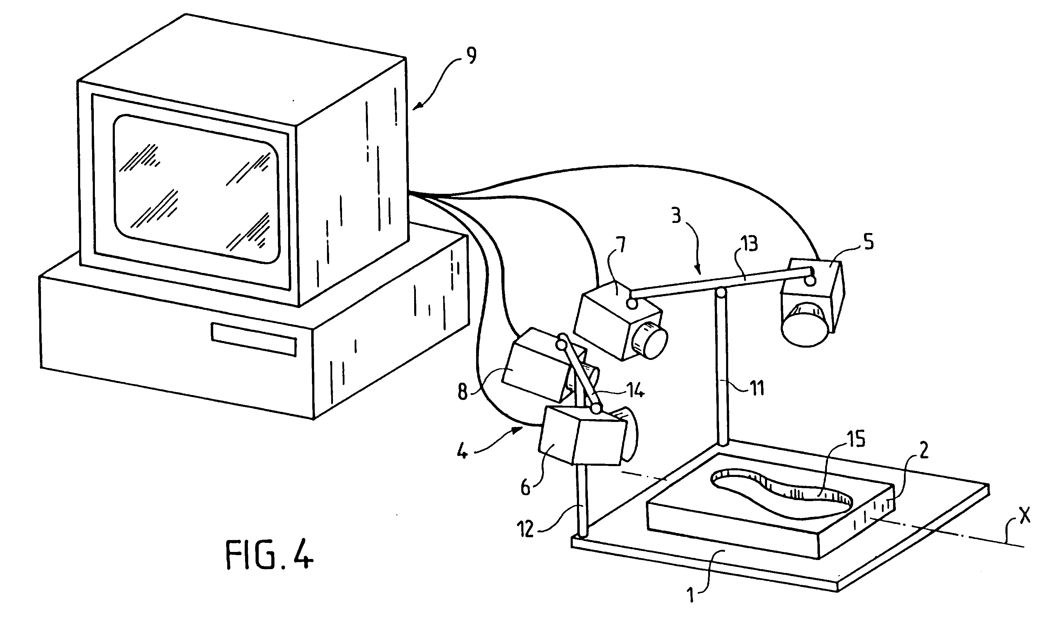

La figure 4 est identique à la figure 3 en ce qui concerne l'emplacement des capteurs. Le pied a été retiré du support déformable 12 pour ne laisser qu'une empreinte 15 et les deux caméras réalisent la mesure de l'empreinte.

Que la mesure globale d'un pied s'effectue selon les figures 1 et 2 ou 3 et 4, le calculateur regroupe toutes les mesures pour déterminer l'enveloppe globale du pied au moyen de multiples points déterminés chacun par trois coordonnées. Tous ces points sont enregistrés sur un support, par exemple magnétique, qui servira de base au calcul de la forme adéquate et de sa fabrication. Des données en trois dimensions de cette forme, on peut alors en déduire le patronage, c'est-à-dire la découpe des pièces de cuir nécessaires à la fabrication de chaussures.

Bien entendu, l'appareil peut comporter un troisième capteur (par exemple le capteur 4 de la figure 1 rajouté aux deux capteurs de la figure 3), voire un quatrième capteur, ce qui augmentera la précision de mesure ou permettra de mesurer des formes plus complexes, telles qu'on les rencontre notamment dans certaines pathologies du pied.

Dans le cas de la figure 3, l'appareil peut aussi comporter deux capteurs que l'on déplace chacun selon un axe parallèle à l'axe X, si on utilise des capteurs qui nécessitent une translation, ou encore un seul capteur monté sur une seule potence, réalisant un mouvement de translation tantôt du côté interne, tantôt du côté externe du pied.

Tout capteur de forme sans contact peut être utilisé.

## Revendications

1. Procédé de mesure tridimensionnelle, sans contact, de l'enveloppe d'une partie (10) du corps d'une personne, en particulier un pied, par digitalisation à l'aide d'un moyen de capture (3,4) relié à un calculateur (9), caractérisé en ce qu'il consiste à poser ladite partie sur un support déformable rémanent (2) permettant de supporter ladite partie en charge, à mesurer la partie en deux temps, un premier temps correspondant à la mesure de la région supérieure de la partie par ledit moyen de capture occupant une première position repérable par rapport au support et, après avoir retiré la partie du support, un deuxième temps correspondant à la mesure de l'empreinte (15) de la partie sur le même support par ledit moyen de capture occupant une deuxième position également repérable par rapport à ce même support, les deux positions du moyen de capture étant identiques ou différentes, les deux mesures étant alors traitées pour reconstituer l'enveloppe globale de ladite partie.

2. Procédé selon la revendication 1, caractérisé en ce qu'il consiste à effectuer au moins deux prises de vue pour le premier temps de mesure et au moins une prise de vue pour le deuxième temps de mesure.

3. Procédé selon la revendication 2, caractérisé en ce que les deux prises de vue pour le premier temps de mesure sont une prise de vue postérieure, supérieure et axiale, et une prise de vue antérieure, supérieure et axiale.

4. Procédé selon la revendication 2, caractérisé en ce que les deux prises de vue pour le premier temps de mesure sont une prise de vue postérieure, supérieure et latérale et une prise de vue postérieure, supérieure et médiale.

5. Procédé selon la revendication 4, caractérisé en ce qu'il consiste à effectuer une troisième prise de vue antérieure, supérieure et axiale.

6. Procédé de mesure selon la revendication 2, caractérisé en ce que la prise de vue pour le deuxième temps de mesure est une prise de vue supérieure, axiale, antérieure ou postérieure.

7. Procédé de mesure selon la revendication 2, caractérisé en ce que le deuxième temps de mesure s'effectue selon deux prises de vue supérieure, axiale, l'une antérieure et l'autre postérieure.

8. Procédé de mesure selon la revendication 2, caractérisé en ce que le deuxième temps de mesure s'effectue selon deux prises de vue, une prise de vue postérieure, supérieure et latérale et une prise de vue postérieure, supérieure et médiale.

9. Appareil de mesure tridimensionnelle sans contact de l'enveloppe d'une partie du corps humain, en particulier un pied d'une personne, par digitalisation, à l'aide d'un moyen de capture (3,4) relié à un calculateur (9), caractérisé en ce qu'il comporte un support déformable rémanent (2) recevant ladite partie à mesurer en charge (10), et en ce que le moyen de capture (3,4) occupe au moins une position repérable par rapport au support (2).

10. Appareil selon la revendication 9, caractérisé en ce que le moyen de capture comporte un projecteur (7,8) et une caméra (5,6) qui forment entre eux un angle déterminé, ce projecteur et cette caméra étant placés l'un par rapport à l'autre à une distance déterminée.

11. Appareil selon la revendication 10, caractérisé en ce que le moyen de capture comporte un capteur déplaçable pour effectuer des prises de vue d'au moins deux points de vue différents.

12. Appareil selon la revendication 10, caractérisé en ce que le moyen de capture comporte plusieurs capteurs fixes dans l'espace.

13. Appareil selon la revendication 12, caractérisé en ce qu'il comporte deux capteurs situés dans l'axe du support effectuant, l'un une prise de vue postérieure et supérieure, tandis que l'autre effectue une prise de vue antérieure et supérieure.

14. Appareil selon la revendication 12, caractérisé en ce qu'il comporte deux capteurs placés de manière désaxée par rapport à l'axe du support et effectuant, l'un une prise de vue postérieure, supérieure et latérale, et l'autre une prise de vue postérieure, supérieure et médiale.

## Patentansprüche

1. Die hiesige Erfindung betrifft ein dreidimensionales Verfahren für die berührungsfreie Messung der Hülle eines menschlichen Körperteils (10) und insbesondere des Fußes durch Digitalisierung mit Hilfe eines an einen Rechner (9) angeschlossenen Kaptorensystems (3, 4), welches dadurch gekennzeichnet ist, daß es darin besteht, den besagten Körperteil auf einen verformbaren remanenten Untergrund (2) zu stellen, der diesen Körperteil im belasteten Zustand trägt und dann den Körperteil in zwei Phasen zu messen, wobei der erste Messung die obere Region des betreffenden Körperteils durch die besagte Erfassung einer ersten Position im Verhältnis zum Träger betrifft und die zweite Messung nach dem Entfernen des Trägerteils den Abdruck (15) des Körperteils auf demselben Träger durch das besagte Kaptorensystems an der zweiten Position betrifft, welche ebenfalls im Verhältnis zu demselben Träger erfaßbar ist. Die beiden Positionen für die Erfassung sind dabei identisch oder verschieden und die beiden Messungen werden verarbeitet, um die gesamte Hülle des besagten Körperteils zu rekonstruieren.

2. Verfahren gemäß der Anmeldung 1, welches dadurch gekennzeichnet ist, daß es darin besteht, daß mindestens zwei Aufnahmen für die erste Messungsphase und mindestens eine Aufnahme für die zweite Messungsphase gemacht werden.

3. Verfahren gemäß der Anmeldung 2, welches dadurch gekennzeichnet ist, daß die beideii Aufnahmen für die erste Messungsphase aus einem hinteren, oberen und axialen Blickwinkel und eine Aufnahme aus einem vorderen, oberen und axialen Blickwinkel gemacht werden.

4. Verfahren gemäß der Anmeldung 2, welches dadurch gekennzeichnet ist, daß die beiden Aufnahmen für die erste Messungsphase aus einem hinteren, oberen und seitlichen Blickwinkel und eine Aufnahme aus einem hinteren, oberen und medialen Blickwinkel gemacht werden.

5. Verfahren gemäß der Anmeldung 4, welches dadurch gekennzeichnet ist, daß es darin besteht, eine dritte hintere, obere und axiale Aufnahme zu machen.

6. Meßverfahren gemäß der Anmeldung 2, welches dadurch gekennzeichnet ist, daß die Aufnahme für die zweite Messungsphase aus einem oberen, axialen, vorderen oder hinteren Blickwinkel gemacht wird.

7. Meßverfahren gemäß der Anmeldung 2, welches dadurch gekennzeichnet ist, daß die beiden Aufnahmen für die zweite Messungsphase aus einem oberen, axialen, einerseits vorderen und andererseits hinteren Blickwinkel gemacht werden.

8. Meßverfahren gemäß der Anmeldung 2, welches dadurch gekennzeichnet ist, daß die beiden Aufnahmen für die zweite Messungsphase aus einer Aufnahme aus einem hinteren, oberen und seitlichen Blickwinkel und einer Aufnahme aus einem hinteren, oberen und medialen Blickwinkel bestehen.

9. Dreidimensionales Meßgerät für die berührungsfreie Messung der Hülle eines menschlichen Körperteils, insbesondere der Füße, durch Digitalisierung mit Hilfe eines an einen Rechner (9) angeschlossenen Kaptorensystems (3, 4), welches dadurch gekennzeichnet ist, daß es einen verformbaren remanenten Untergrund (2) umfaßt, auf den der zu messende Körperteil im belasteten Zustand gestellt wird (10) und daß das Kaptorensystem (3, 4) mindestens eine ermittelbare Position im Verhältnis zum Träger (2) hat.

10. Gerät gemäß der Anmeldung 9, welches dadurch gekennzeichnet ist, daß es eine Erfassungsausrüstung umfaßt, die aus einem Projektor (7, 8) und einer Kamera (5, 6) besteht, die einen bestimmten Winkel zueinander bilden und wobei dieser Projektor und diese Kamera auf einen bestimmten Abstand voneinander aufgestellt sind.

11. Gerät gemäß der Anmeldung 10, welches dadurch gekennzeichnet ist, daß das Erfassungssystem einen verstellbaren Kaptor für Aufnahmen aus wenigstens zwei verschiedenen Blickwinkeln umfaßt.

12. Gerät gemäß der Anmeldung 10, welches dadurch gekennzeichnet ist, daß das Erfassungssystem mehrere räumlich feste Kaptoren umfaßt.

13. Gerät gemäß der Anmeldung 12, welches dadurch gekennzeichnet ist, daß es zwei Kaptoren umfaßt, die sich in der Achse des Trägers befinden, wobei der erste Kaptor eine hintere und obere Aufnahme macht, während der zweite Kaptor eine vordere und obere Aufnahme macht.

14. Gerät gemäß der Anmeldung 12, welches dadurch gekennzeichnet ist, daß es zwei Kaptoren umfaßt, die sich außerhalb der Achse des Trägers befinden und die einerseits eine hintere, obere und seitliche Aufnahme und andererseits eine hintere, obere und mediale Aufnahme machen.

## Claims

1. A contact free three dimensional system for measuring the external form of a part (10) of the human body, in particular the foot, through digitisation using an acquisition system (3,4) linked to a computer (9), distinguished by the fact that it consists in the positioning of said part on a compression set base (2) which supports the aforesaid part under load, for the purpose of measuring the part in two phases, the first phase being the measurement of the upper region of the part using the aforesaid acquisition system in an initial position which is indexable in relation to the support and, after having removed the put from the support base, a second phase which covers the measurement of the imprint (15) left by the aforesaid part on the aforesaid support by means of the aforesaid acquisition system in a second position which is also indexable in relation to the same support, both positions of the acquisition system being identical or different, both measurements being processed in order to reproduce the overall shape of said put.

2. System according to claim 1, distinguished by the fact that it consists in taking at least two photographs for the first measurement phase and at least one photograph for the second measurement phase.

3. System according to claim 2, distinguished by the fact that the two photographs taken for the first measurement phase comprise one posterior, superior and axial photograph, and one anterior, superior and axial photograph.

4. System according to claim 2, distinguished by the fact that the two photographs for the first measurement phase comprise a posterior, superior and lateral photograph and a posterior, superior and medial photograph.

5. System according to claim 4, distinguished by the fact that it consists in taking a third photograph which is anterior, superior and axial.

6. Measurement system according to claim 2, distinguished by the fact that the second measurement phase photograph consists in a superior, axial, anterior or posterior photograph.

7. Measurement system according to claim 2, distinguished by the fact that the second measurement phase takes the form of two superior, axial photographs, one being anterior and the other posterior.

8. Measurement system according to claim 2, distinguished by the fact that the second measurement phase consists of two photographs, one posterior, superior and lateral and one posterior, superior and medial.

9. A three dimensional contact free apparatus for measuring the external form of parts of the human body, in particular a human foot, through digitisation, using a means of acquisition (3,4) connected to a computer (9), distinguished by the fact that it consists of a compression set base (2) supporting said part to be measured under load (10), and where the acquisition system (3,4) takes up at least one position which can be indexed in relation to the support (2).

10. Apparatus according to claim 9, distinguished by the fact that the acquisition system comprises a projector (7,8) and a camera (5,6) which between them form a specific angle, said projector and said camera being separated by a specific distance.

11. Apparatus according to claim 10, distinguished by the fact that the acquisition system consists of a moving sensor used to take photographs from at least two different view points.

12. Apparatus according to claim 10, distinguished by the fact that the acquisition system consists of several sensors which are fixed in space.

13. Apparatus according to claim 12, distinguished by the fact that it consists of two sensors located in the axis of the support base and one of which takes a posterior and superior photograph, whereas the other takes an anterior and superior photograph.

14. Apparatus according to claim 12, distinguished by the fact that it consists of two sensors located off-centre in relation to the support axis, one taking a posterior, superior and lateral photograph, and the other a posterior, superior and medial photograph.
